(19) Europäisches Patentamt
European Patent Office
Office européen des brevets

(11) Numéro de publication: **0 093 840**
**B1**

# (12) FASCICULE DE BREVET EUROPEEN

(45) Date de publication du fascicule du brevet: **22.05.85**

(21) Numéro de dépôt: **83101531.8**

(22) Date de dépôt: **18.02.83**

(51) Int. Cl.[4]: **C 07 C 45/00,** C 07 C 49/04, C 07 C 49/205, C 07 C 49/29, C 07 C 49/527, C 07 C 49/557, A 61 K 7/46

## (54) Procédé pour la préparation de composés cétoniques.

(30) Priorité: **20.04.82 CH 2375/82**

(43) Date de publication de la demande:
**16.11.83 Bulletin 83/46**

(45) Mention de la délivrance du brevet:
**22.05.85 Bulletin 85/21**

(84) Etats contractants désignés:
**CH DE FR GB LI NL**

(56) Documents cités:
**US - A - 3 059 032**

**Chemical Abstracts vol. 97, no. 19, 8 novembre 1982, Columbus, Ohio, USA page 666, colonne 2, abstract no. 162450b**
**Chemical Abstracts vol. 78, no. 21, 28 mai 1973, Columbus, Ohio, USA F. HUET et al. "Formation of ketonic enolates by the action of alkylmagnesium halides on esters in hexamethylphosphoric triamide. Direction of the enolization and the preparation of ketones", page 300-310, abstract no. 135245q**

(73) Titulaire: **FIRMENICH SA, 1, route des Jeunes, CH-1211 Genève 8 (CH)**

(72) Inventeur: **Fehr, Charles, Dr., 6, chemin Ravoux, CH-1290 Versoix (CH)**

(74) Mandataire: **Salvadori, Giuseppe, Dr., c/o Firmenich S.A. Case Postale 239, CH-1211 Genève 8 (CH)**

EP 0 093 840 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La formation directe de cétones par une réaction du type de Grignard sur des esters carboxyliques, dans l'éther ou dans un hydrocarbure, est généralement considérée comme une réaction peu satisfaisante, notamment à cause de la formation d'alcools tertiaires non désirés [«Synthesis», 877 (1980)]. Ce type de réaction a été appliqué sur des esters aliphatiques dans l'hexaméthylphosphorotriamide (HMPT) pour fournir les cétones correspondantes. Elle a été décrite par F. Huet *et al.* dans «Tetrahedron», *29*, 479 (1973); son application à l'échelle industrielle se heurte cependant à deux problèmes majeurs: la cancérigénité et la disponibilité limitée de l'HMPT. Cette méthode s'est en outre révélée fort peu satisfaisante dans les essais de transformation des esters dérivés de l'acide benzoïque ou des esters aliphatiques α-, β-insaturés et aromatiques. Les rendements en cétones obtenues sont dans ce cas modestes, peut-être à cause de la faible énolisation des cétones intermédiaires formées au cours de la réaction.

Compte tenu de l'importance des composés cétoniques dans des domaines variés, ce type de réaction cependant présente un grand potentiel industriel.

Parmi le nombre de composés cétoniques pouvant présenter un intérêt pour l'industrie de la parfumerie et des arômes figurent notamment plusieurs cétones cycloaliphatiques dont l'utilisation au cours des dernières années n'a cessé de s'étendre. Il s'agit tout particulièrement des damascones et des damascénones ainsi que de leurs dérivés (voir par exemple le brevet CH No 520479 et le brevet RFA DE-PS No 2022216). Nombreuses ont été à ce jour les publications relatives à des procédés pour leur fabrication.

Le brevet CH No 563951 décrit un procédé qui consiste en la pyrolyse d'un alcool allylique tertiaire de formule:

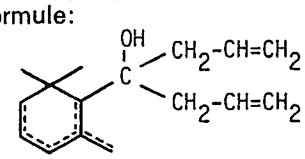

En pratique, un tel procédé s'est révélé d'une application industrielle peu aisée, surtout à cause de la formation de produits secondaires qui, en présentant une odeur désagréable, peuvent altérer le caractère organoleptique propre des produits désirés. Les alcools allyliques tertiaires susmentionnés peuvent être précisément préparés par une réaction du type de Grignard sur des esters de formule:

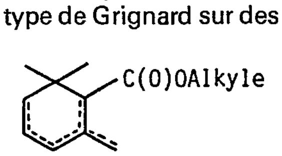

conformément à la méthode décrite par exemple dans «Helv. Chim. Acta», *54*, 1774 (1971). Une telle addition ne s'arrête malheureusement pas à la formation de la cétone, mais conduit normalement aux alcools allyliques tertiaires précités, d'où la nécessité de procéder à leur transformation ultérieure par voie de pyrolyse afin d'obtenir les dérivés de la damascone ou damascénone désirés.

Nous avons maintenant découvert qu'il était désormais possible de préparer ces derniers par une addition directe d'un halogénomagnésien sur un amide ou un ester approprié, pour autant qu'on effectue cette addition en présence d'au moins 1 Eq d'une base forte constituée par un amidure de métal alcalin.

Le choix particulier pour une telle base doit satisfaire à trois critères principaux: exercer une bonne stabilité sur l'énolate qui se forme en cours de réaction, avoir un pouvoir déprotonant élevé et enfin être peu nucléophilique. Un tel procédé peut par ailleurs s'appliquer à une grande variété d'esters, et non seulement à ceux de type cycloaliphatique. De préférence cependant, il s'applique à la transformation d'esters de formule:

R–C(O)OAlkyle

dans laquelle le symbole R désigne un radical alkyle dont le carbone lié au groupe carbonyle est soit tétrasubstitué, soit stériquement encombré.

La présente invention a donc pour objet un procédé pour la préparation de composés carbonylés de formule:

$$\begin{array}{c} R' \quad O \\ | \quad \; \| \\ R''-C-C-R^1 \\ | \qquad \\ R''' \qquad \end{array} \qquad (I)$$

dans laquelle les symboles R', R'' et R''', identiques ou différents, pris séparément, représentent chacun un radical alkyle linéaire ou ramifié, où R' et R'', pris conjointement avec le carbone auquel ils sont liés, représentent un radical cycloaliphatique, substitué ou non, saturé ou insaturé, et R''' désigne un radical alkyle ou hydroxyle ou un atome d'hydrogène, et $R^1$ sert à désigner un radical alkyle, saturé ou insaturé, lequel procédé est caractérisé en ce qu'on traite, dans un milieu organique inerte, en présence d'au moins 1 Eq d'une base forte constituée par un amidure de métal alcalin, un composé de formule

$$\begin{array}{c} R' \quad O \\ | \quad \; \| \\ R''-C-C-Z \\ | \qquad \\ (R''')_n \qquad \end{array} \qquad (II)$$

dans laquelle

a. R', R'' et R''', lorsqu'ils sont pris séparément, ont le sens indiqué ci-dessus, n valant 1, ou

b. R' et R'', lorsqu'ils sont pris conjointement avec le carbone auquel ils sont liés, représentent un radical cycloaliphatique, substitué ou non, saturé ou insaturé, et R''' désigne un radical alkyle ou un atome d'hydrogène, n valant 1, ou

c. R' et R'', lorsqu'ils sont pris conjointement avec le carbone auquel ils sont liés, représentent un radical monovalent de formule:

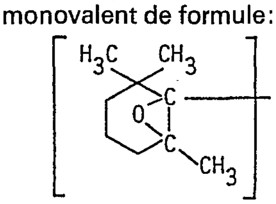

n valant 0, et

Z représente soit un groupe alkoxyle, soit un radical de formule $-N(R)_2$, dans laquelle R désigne un radical alkyle, avec un halogénomagnésien de formule:

$$R^1-MgC$$

dans laquelle $R^1$ a le sens indiqué plus haut et X représente un atome d'halogène.

La réaction s'effectue dans un milieu constitué par un solvant ou un mélange de solvants organiques inertes. A cet effet, on emploie des solvants tels que des éthers, comme l'éther diéthylique ou le tétrahydrofuranne.

A titre de base forte, on utilise un amidure d'un métal alcalin, de préférence un amidure de lithium. C'est ainsi que des bases telles que le diméthyl-, le diéthyl- ou le diisopropylamidure de lithium constituent des bases préférentielles. Des mélanges de deux des bases précitées peuvent également être employés.

Comme indiqué plus haut, ladite base est utilisée à raison d'au moins 1 Eq par rapport au produit de départ (II). Nous avons cependant remarqué que, en opérant avec des quantités supérieures à 1 Eq, on pouvait augmenter sensiblement la sélectivité de la réaction en diminuant la formation des produits issus de la double addition du magnésien sur le produit de départ.

Suivant un mode préférentiel d'exécution du procédé de la présente invention, on peut opérer en présence de 1 Eq de diéthyl- ou de diméthylamidure de lithium et de 1 Eq de diisopropylamidure de lithium. C'est ainsi, par exemple, qu'on a pu transformer le 1,3- et le 1,4-diméthylcyclohex-3-énylcarboxylate de méthyle en 1,3- et 1,4-diméthylcyclohex-3-énylallylcétone. Cette même cétone est obtenue, suivant le procédé de l'invention, à partir de l'amide correspondant, à savoir le N,N-diéthyl-1,3- ou le N,N-diéthyl-1,4-diméthylcyclohex-3-énylcarboxamide de formule:

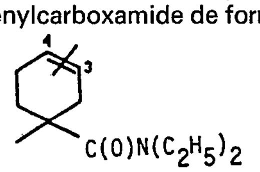

Il est intéressant de remarquer l'effet exercé par la présence d'une base forte telle que le diisopropylamidure de lithium sur la sélectivité de la réaction de transformation du 1,3- ou 1,4-diméthylcyclohex-3-énylcarboxylate de méthyle. Le tableau 1 résume les résultats expérimentaux observés.

*Tableau 1*

| Produit de départ | Réactif | Equiv. LDA[1] | Produits finals | | |
|---|---|---|---|---|---|
| 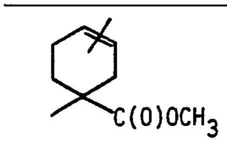 | 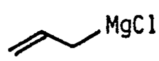 | | 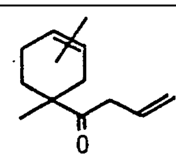 | | 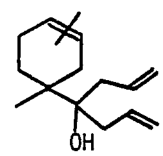 |
| | | 3 | 2 | : | 1 |
| | | 0 | 1 | : | 7 |

[1] Diisopropylamidure de lithium.

En effectuant cette même réaction à l'aide de diéthylamidure de lithium ou un mélange de cette même base avec du diisopropylamidure de lithium, on a observé une meilleure sélectivité. Le tableau 2 illustre ce fait.

*Tableau 2*

| Produit de départ | Réactif | Equiv. base | Produits finals | | |
|---|---|---|---|---|---|
| 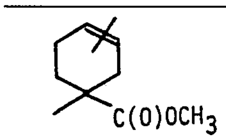 | 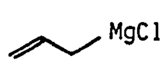 | | 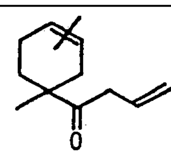 | | 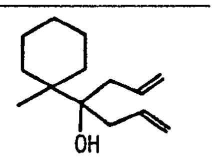 |
| | 2 Eq | 2,2 $LINEt_2$[1] | 92 | : | 8 |
| 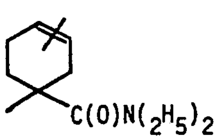 | 1,1 Eq | 1 $LINEt_2$[1]<br>1 LDA[2] | 89 | : | 11 |
| | 1,1 Eq | 1 LDA[2] | 92 | : | 8 |
| | 1,6 Eq | 0 | 40 | : | 60 |

[1] Diéthylamidure de lithium.
[2] Diisopropylamidure de lithium.

Les bases utilisées peuvent être obtenues, à l'aide d'un procédé connu, à partir de styrène ou de méthylstyrène suivant la réaction décrite par F. Gaudemar-Bordone *et al.* [«Synthesis», *1979*, 463] et M.T. Reetz *et al.* [«Ann.», *1980*, 1471].

Pour plus de compréhension, le déroulement des différentes opérations qui caractérisent le procédé de l'invention est illustré à l'aide du schéma donné ci-dessous (l'ordre d'adjonction est indiqué par les chiffres 1 à 3).

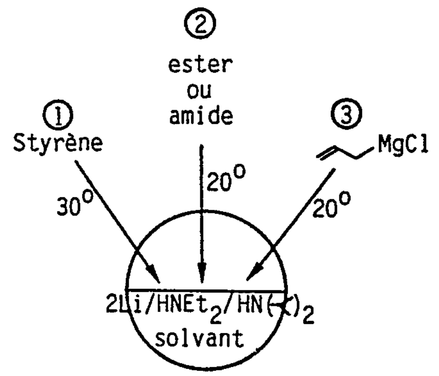


Comme indiqué plus haut, le procédé peut s'effectuer à une température comprise entre −70 et +65°C, mais de préférence à température ambiante ou à une température voisine de celle-ci. Au point de vue de l'économie d'énergie, ce fait représente en soi un avantage industriel certain.

Le procédé de l'invention peut donc s'appliquer à une grande variété d'esters et d'amides, notamment cycloaliphatiques. En effectuant la réaction sur le 1,2-époxy-2,6,6-triméthylcyclohexylcarboxylate de méthyle, le composé de formule:

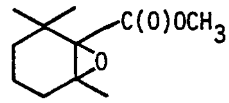

on obtient un carbinol de formule:

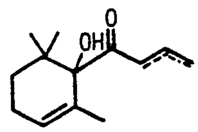 ou de formule: 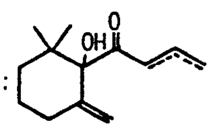

contenant une double liaison dans les positions indiquées par les pointillés, lesquels composés peuvent être transformés en β-damascénone par un traitement à l'aide d'un agent acide.

L'obtention de l'un ou de l'autre des carbinols cités est déterminée par le choix de la base. En effectuant la réaction en présence de 2 Eq de diéthylamidure de lithium en mélange avec 1 Eq de diisopropylamidure de lithium, on obtient le carbinol de type α. Par contre, en effectuant la réaction en présence de 2 Eq de diisopropylamidure de lithium, on obtient l'isomère γ correspondant. Ce fait peut être illustré par le schéma réactionnel que voici:

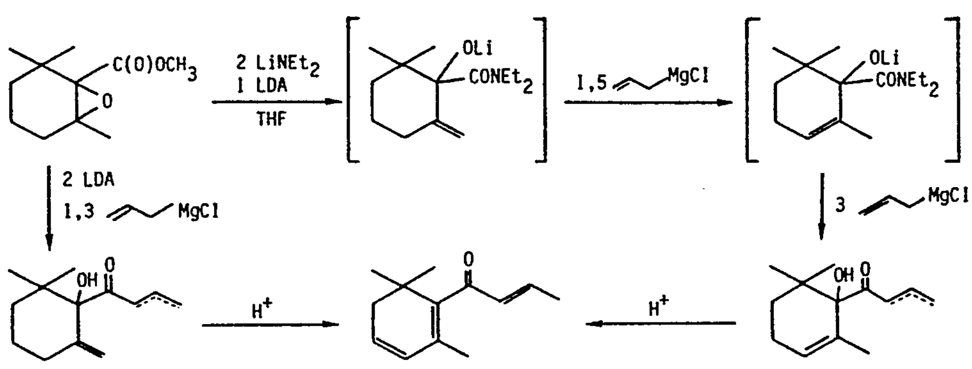


γ- α-

Le carbinol γ ainsi obtenu, ou 6,6-diméthyl-2-méthylène-1-crotonoyl-1-hydroxycyclohexane, est un composé nouveau qui présente des propriétés olfactives fort intéressantes et qui, de ce fait, peut être utilisé en tant qu'ingrédient parfumant.

L'invention est illustrée à l'aide des exemples suivants dans lesquels les températures sont indiquées en degrés centigrades et les abréviations ont le sens usuel dans l'art.

*Exemple 1:*

*Méthode A*

Dans un réacteur tricol équipé d'une entrée d'azote, d'un thermomètre et d'un agitateur magnétique, on introduit 1,1 Eq de diisopropylamine préalablement distillée sur du KOH, 1,1 Eq de diéthylamine, également distillée sur du KOH, et du tétrahydrofuranne (THF) anhydre. Puis on introduit rapidement sous azote une solution de butyllithium dans l'hexane d'une molarité prédéterminée en veillant à ce que la température du mélange de réaction ne dépasse pas 35°. La formation complète de diisopropyl- et diéthylamidure de lithium a lieu en 30 min environ. A la même température, on introduit goutte à goutte dans la solution formée l'ester (1,0 Eq) de départ.

Une solution de chlorure d'allylmagnésium dans le RHF (1,1 Eq) est ensuite ajoutée en 3 min environ au mélange de réaction maintenu à une température inférieure à 25°. Après 10 min, le mélange est hydrolysé en le versant dans une solution saturée de $NH_4Cl$ et de glace.

Après extraction à l'acétate d'éthyle, lavage des extraits organiques réunis, séchage et évaporation des parties volatiles, on obtient le produit brut qui peut être purifié à l'aide de chromatographie sur $SiO_2$ ou par recristallisation.

*Méthode B*

Le même appareillage que celui utilisé pour la méthode A a été employé pour la préparation d'une solution de 2 Eq de diisopropylamidure de lithium. Le chlorure d'allylmagnésium dans le THF (1,3 Eq) y a été ajouté en une seule portion, suivi goutte à goutte de l'ester de départ (1,0 Eq). On observe un réchauffement du mélange réactionnel ainsi que la formation d'une suspension jaune. La température est maintenue à environ 25° et le produit est isolé suivant la méthode A.

*Méthode C*

Le même appareillage que celui utilisé pour la méthode A a été employé pour la préparation d'une solution de 2,5 mmol de diisopropylamidure de lithium. 1,0 Eq de l'époxyester de départ est rapidement ajouté à la solution de base obtenue. Cette addition s'accompagne d'un fort dégagement de chaleur, ce qui entraîne l'ébullition du THF présent. Après 15 min, on ajoute goutte à goutte le chlorure d'allylmagnésium (1,3 Eq) dans le THF et il se forme alors une suspension jaune qui est hydrolysée après 10 min dans une solution de chlorure d'ammonium et de glace. L'isolation du produit a lieu comme indiqué à la méthode A.

*α-, β-Isodamascone et β-isodamascénone*

En appliquant la méthode B, on a pu préparer de l'α- et β-isodamascone ainsi que de la β-isodamascénone. Ces produits sont obtenus en mélange avec une quantité mineure de leur isomère α- et β-damascone et β-damascénone. Les mélanges obtenus peuvent être soumis directement à acidification pour fournir ces derniers composés exclusivement.

*1-Hydroxy-γ-isodamascone* 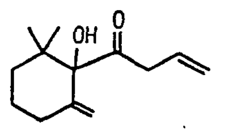

Ce composé est obtenu en suivant la méthode C à partir de 1,2-époxy-2,6,6-triméthylcyclohexylcarboxylate de méthyle (600 mg).

L'huile jaune obtenue (612 mg) a été purifiée par chromatographie sur $SiO_2$ (éluant: cyclohexane/acétate d'éthyle 95/5). On a ainsi obtenu 437 mg du produit désiré en mélange, à raison de 95/5, avec l'hydroxycétone conjuguée; p.f.: 62-63°.

IR (film, liquide): 3560, 2950, 1720, 1640, 1460, 1390, 1310, 1080 et 920 $cm^{-1}$;

RMN (60 MHz): 0,90 (3H, s); 0,97 (3H, s); 1,20-1,90 (3H, m); 2,12 (1H, s); 2,20-2,70 (3H, m); 3,47 (2H, d, J≃7 Hz); 4,80-5,50 (4H, m); 5,50-6,40 (1H, m) δ ppm;

SM: $M^+$=208(<1); m/e: 139(14), 95(31), 69(9), 55(8), 43(100), 40(18), 38(5).

*1-Hydroxy-α-damascone* 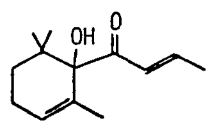

Ce composé est obtenu suivant la méthode A à partir de 1,2-époxy-2,6,6-triméthylcyclohexylcarboxylate de méthyle (600 mg). Cet ester a été introduit rapidement dans le mélange de base et l'on a observé un réchauffement de celui-ci à 30° environ ainsi que la formation d'un précipité. Le mélange a été ensuite chauffé à environ 50° et, à cette même température, on y a ajouté 3 Eq d'une solution de chlorure d'allylmagnésium.

Le produit a été isolé comme indiqué à la méthode A et une purification par distillation a permis d'obtenir 193 mg du produit désiré.

*1,3- et 1,4-Diméthyl-cyclohex-3-énylallylcétone*

Ce composé a été obtenu suivant la méthode A à partir d'un mélange de 1,3- et 1,4-diméthylcyclohex-3-énylcarboxylate de méthyle.

A partir de 1,68 g (10 mmol) de ce mélange, on a obtenu 1,80 g d'une huile jaune qui, après distillation, a fourni 1,34 g d'un mélange 92/8 du produit désiré et de l'isomère conjugué correspondant. Eb. environ 80°/1,33 Pa.

L'amide intermédiaire, ou N,N-diéthyl-1,3- et N,N-diéthyl-1,4-diméthylcyclohex-3-énylcarboxamide, a pu être isolé avec un rendement de 90% après purification par distillation. Ses caractères analytiques étaient les suivants: Eb. environ 120°/4,66×$10^2$ Pa;

IR (film, liquide): 2950, 1630, 1420, 1280, 1220, 1150, 1100, 1070 $cm^{-1}$;

RMN (60 MHz): 1,15 (6H, t, J≃7 Hz); 1,23 (3H, s); 1,55-2,27 (9H, m); 3,42 (4H, q, J≃7 Hz); 5,20-5,50 (3H, m) δ ppm;

SM: $M^+$=209(51); m/e: 194(23), 140(13), 109(88), 108(100), 102(18), 100(61), 93(47), 81(11), 72(56), 68(32), 58(16), 45(26), 41(36).

Le N,N-diméthyl-1,3- et N,N-diméthyl-1,4-diméthylcyclohex-3-énylcarboxamide a été obtenu par la même méthode à partir du même ester au moyen de 2 Eq de diméthylamidure de lithium selon la méthode A. Le produit ainsi obtenu possède les caractères analytiques suivants:

Eb. environ 100°/5,32×$10^2$ Pa;

IR (film, liquide): 2950, 1630, 1500, 1450, 1390, 1260, 1150, 1100 et 1060 $cm^{-1}$;

RMN (60 MHz): 1,29 (3H, s); 1,57-2,23 (9H, m); 3,02 (6H, s); 5,20-5,52 (3H, m) δ ppm;

SM: $M^+$=181(32); m/e: 109(100), 108(37), 93(23), 72(40), 66(27), 41(14).

**Revendications**

1. Procédé pour la préparation de composés carbonylés de formule:

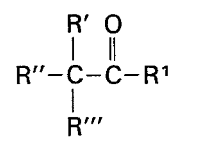 (I)

dans laquelle les symboles R', R'' et R''', identiques ou différents, pris séparément, représentent chacun un radical alkyle linéaire ou ramifié, ou R' et R'', pris conjointement avec le carbone auquel ils sont liés, représentent un radical cycloaliphatique, substitué ou non, saturé ou insaturé, et R''' désigne un radical alkyle ou hydroxyle ou un atome d'hy-

drogène, et $R^1$ sert à désigner un radical alkyle, saturé ou insaturé, lequel procédé est caractérisé en ce qu'on traite, dans un milieu organique inerte, en présence d'au moins 1 Eq d'une base forte constituée par un amidure de métal alcalin, un composé de formule:

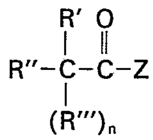 (II)

dans laquelle

a. R', R'' et R''', lorsqu'ils sont pris séparément, ont le sens indiqué ci-dessus, n valant 1, ou

b. R' et R'', lorsqu'ils sont pris conjointement avec le carbone auquel ils sont liés, représentent un radical cycloaliphatique, substitué ou non, saturé ou insaturé, et R''' désigne un radical alkyle ou un atome d'hydrogène, n valant 1, ou

c. R' et R'', lorsqu'ils sont pris conjointement avec le carbone auquel ils sont liés, représentent un radical monovalent de formule:

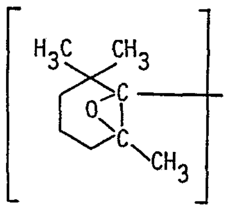

n valant 0, et

Z représente soit un groupe alkoxyle, soit un radical de formule $-N(R)_2$, dans laquelle R désigne un radical alkyle, avec un halogénomagnésien de formule:

$$R^1-MgX$$

dans laquelle $R^1$ a le sens indiqué plus haut et X représente un atome d'halogène.

2. Procédé suivant la revendication 1, caractérisé en ce que la réaction s'effectue dans l'éther ou le tétrahydrofuranne.

3. Procédé suivant la revendication 1, caractérisé en ce que l'on utilise comme base forte le diméthyl-, le diéthyl-, ou le diisopropylamidure de lithium, ou un mélange de deux desdites bases.

4. Procédé suivant la revendication 1, caractérisé en ce que la réaction s'effectue à une température comprise entre −70 et +65° C.

5. Procédé suivant l'une des revendications 1 à 4, caractérisé en ce qu'on utilise comme composé de formule (II) le 1,3- ou 1,4-diméthylcyclohex-3-énylcarboxylate de méthyle pour obtenir la 1,3- ou 1,4-diméthylcyclohex-3-énylallylcétone.

6. Procédé suivant l'une des revendications 1 à 4, caractérisé en ce qu'on utilise, comme composé de formule (II), le N,N-diméthyl- ou le N,N-diéthyl-1,3- ou -1,4-diméthylcyclohex-3-énylcarboxamide pour fournir la 1,3- ou 1,4-diméthylcyclohex-3-énylallylcétone.

7. Procédé suivant la revendication 6, caractérisé en ce qu'on utilise, comme base forte, au moins 1 Eq de diisopropylamidure de lithium.

8. Procédé suivant l'une des revendications 1 à 4, caractérisé en ce que l'on utilise, comme composé de formule (II), le 2,6,6-triméthylcyclohex-2-énylcarboxylate de méthyle pour fournir l'α-damascone.

9. Procédé suivant l'une des revendications 1 à 4, caractérisé en ce qu'on utilise, comme composé de formule (II), le 2,6,6-triméthylcyclohex-1-énylcarboxylate de méthyle pour fournir la β-damascone.

10. Procédé suivant l'une des revendications 1 à 4, caractérisé en ce qu'on utilise, comme composé de formule (II), le safranate de méthyle pour fournir la β-damascénone.

11. Procédé suivant les revendications 1 à 4, caractérisé en ce qu'on utilise, comme composé de formule (II), le 1,2-époxy-2,6,6-triméthylcyclohexylcarboxylate de méthyle pour fournir, à l'aide de 2 Eq de diisopropylamidure de lithium comme base, la 1-hydroxy-γ-isodamascone.

## Claims

1. Process for the preparation of carbonyl compounds of formula:

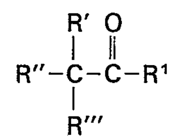 (I)

wherein each of symbols R', R'' and R''', identical or different, represents, when taken separately, a linear or branched alkyl radical, or R' and R'', when taken together with the carbon atom to which they are bound, represent a substituted or unsubstituted, saturated or unsaturated cycloaliphatic radical, and R''' designates an alkyl radical, a hydroxyl or a hydrogen atom, and $R^1$ represents a saturated or unsaturated alkyl radical, which process is characterized in that a compound of formula:

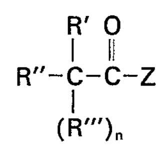 (II)

wherein

a. R', R'' and R''', when taken separately, have the above-given meaning, n standing for 1, or

b. R' and R'', when taken together with the carbon atom to which they are bound, represent a substituted or unsubstituted, saturated or unsaturated cycloaliphatic radical and R''' designates an alkyl radical or a hydrogen atom, n standing for 1, or

c. R' and R'', when taken together with the carbon atom to which they are bound, represent a monovalent radical of formula:

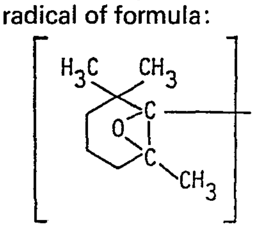

n standing for 0, and

Z represents either an alkoxy group, or a radical of formula $-N(R)_2$, wherein R designates an alkyl radical, is reacted with a magnesium halide of formula:

$$R^1-MgX$$

wherein $R^1$ has the above-indicated meaning and X represents a halogen atom, in an inert organic medium and in the presence of at least an equivalent of a strong base constituted by an alkali metal amide.

2. Process according to Claim 1, characterized in that the reaction is effected in ether or tetrahydrofurane.

3. Process according to Claim 1, characterized in that lithium dimethyl-, diethyl-, or diisopropylamide, or a mixture of two of the said bases, are used as strong base.

4. Process according to Claim 1, characterized in that the reaction is effected at a temperature of between −70 and +65° C.

5. Process according to any of Claims 1 to 4, characterized in that methyl 1,3- or 1,4-dimethylcyclohex-3-enyl-carboxylate are used as compounds of Formula (II) to give 1,3- or 1,4-dimethyl-cyclohex-3-enyl allyl ketone.

6. Process according to any of Claims 1 to 4, characterized in that N,N-dimethyl- or N,N-diethyl-1,3- or -1,4-dimethyl-cyclohex-3-enyl-carboxamide are used as compounds of Formula (II) to give 1,3- or 1,4-dimethyl-cyclohex-3-enyl allyl ketone.

7. Process according to Claim 6, characterized in that there is used as strong base at least one equivalent of lithium diisopropylamide.

8. Process according to any of Claims 1 to 4, characterized in that methyl 2,6,6-trimethylcyclohex-3-enyl-carboxylate is used as compound of Formula (II) to give α-damascone.

9. Process according to any of Claims 1 to 4, characterized in that methyl 2,6,6-trimethylcyclohex-1-enyl-carboxylate is used as compound of Formula (II) to give β-damascone.

10. Process according to any of Claims 1 to 4, characterized in that methyl safranate is used as compound of Formula (II) to give β-damascenone.

11. Process according to Claims 1 to 4, characterized in that methyl 1,2-epoxy-2,6,6-trimethylcyclohexyl-carboxylate is used as compound of Formula (II) to give 1-hydroxy-γ-iso-damascone by means of two equivalents of lithium diisopropylamide as base.

**Patentansprüche**

1. Verfahren zur Herstellung von Carbonylverbindungen der Formel:

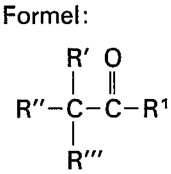

(I)

in welcher jedes der Symbole R′, R″, R‴, identisch oder verschieden, getrennt genommen, ein lineares oder verzweigtes Alkylradikal, oder R′ und R″ zusammen mit dem Kohlenstoff, an welchen sie gebunden sind, ein substituiertes oder unsubstituiertes, gesättigtes oder ungesättigtes cycloaliphatisches Radikal, und R‴ einen Alkyl, Hydroxy- oder Wasserstoffrest darstellen, und $R^1$ ein gesättigtes oder ungesättigtes Alkylradikal bezeichnet, Verfahren welches dadurch gekennzeichnet ist, dass man in einem inerten organischen Milieu in Gegenwart von mindestens einem Äquivalent einer starken Base, welche aus einem Alkalimetallamid besteht, eine Verbindung der Formel:

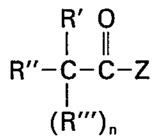

(II)

in welcher

a. R′, R″ und R‴, wenn sie getrennt genommen werden, die oben angeführte Bedeutung haben, wobei n den Wert 1 hat, oder

b. R′ und R″ zusammen mit dem Kohlenstoff, an welchen sie gebunden sind, ein substituiertes oder unsubstituiertes, gesättigtes oder ungesättigtes cycloaliphatisches Radikal darstellen, und R‴ einen Alkyl- oder Wasserstoffrest bezeichnet, wobei n den Wert 1 hat, oder

c. R′ und R″ zusammen mit dem Kohlenstoff, an welchen sie gebunden sind, ein monovalentes Radikal der Formel:

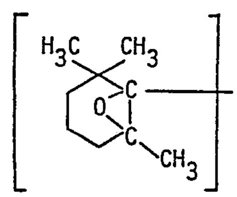

darstellen, wobei n den Wert 0 hat, und

Z entweder eine Alkoxygruppe oder ein Radikal der Formel $-N(R)_2$, in welcher R ein Alkylradikal bezeichnet, darstellt, mit einer Halogen-Magnesium Verbindung der Formel:

$$R^1-MgX$$

in welcher $R^1$ den oben angegebenen Sinn hat und X ein Halogenatom bedeutet, behandelt.

2. Verfahren nach Anspruch 1, dadurch gekennzeichnet, dass die Reaktion in Äther oder Tetrahydrofuran durchgeführt wird.

3. Verfahren nach Anspruch 1, dadurch gekennzeichnet, dass man als starke Base Lithiumdimethyl-, Lithiumdiäthyl- oder Lithiumdiisopropylamid oder ein Gemisch von zwei der oben genannten Basen verwendet.

4. Verfahren nach Anspruch 1, dadurch gekennzeichnet, dass die Reaktion bei einer Temperatur von −70 bis +65° C durchgeführt wird.

5. Verfahren nach einem der Ansprüche 1 bis 4, dadurch gekennzeichnet, dass man als Verbindung der Formel (II) Methyl-1,3- oder 1,4-Dimethylcyclohex-3-enylcarboxylate verwendet, um 1,3- oder 1,4-Dimethylcyclohex-3-enyl-allylketon zu erhalten.

6. Verfahren nach einem der Ansprüche 1 bis 4, dadurch gekennzeichnet, dass man als Verbindung der Formel (II) N,N-Dimethyl- oder N,N-Diäthyl-1,3- oder -1,4-dimethylcyclohex-3-enylcarboxamide verwendet, um 1,3- oder 1,4-Dimethylcyclohex-3-enylallylketon zu erhalten.

7. Verfahren nach Anspruch 6, dadurch gekennzeichnet, dass man als starke Base zumindest ein Äquivalent Lithiumdiisopropylamid verwendet.

8. Verfahren nach einem der Ansprüche 1 bis 4, dadurch gekennzeichnet, dass man als Verbindung der Formel (II) Methyl-2,6,6-Trimethylcyclohex-2-enylcarboxylate verwendet, um α-Damascon zu erhalten.

9. Verfahren nach einem der Ansprüche 1 bis 4, dadurch gekennzeichnet, dass man als Verbindung der Formel (II) Methyl-2,6,6-Trimethylcyclohex-1-enylcarboxylate verwendet, um β-Damascon zu erhalten.

10. Verfahren nach einem der Ansprüche 1 bis 4, dadurch gekennzeichnet, dass man als Verbindung der Formel (II) Methylsafranat verwendet, um β-Damascenon zu erhalten.

11. Verfahren nach einem der Ansprüche 1 bis 4, dadurch gekennzeichnet, dass man als Verbindung der Formel (II) Methyl-1,2-epoxy-2,6,6-trimethylcyclohexylcarboxylate verwendet, um mittels zweier Äquivalente Lithiumdiisopropylamid als Base 1-Hydroxy-γ-isodamascon zu erhalten.